# FASCICULE DE BREVET EUROPEEN

(11) **EP 2 285 325 B1**
(45) Date de publication et mention de la délivrance du brevet: **17.07.2013**
(21) Numéro de dépôt: 09742261.2
(22) Date de dépôt: 07.04.2009
(51) Int. Cl.: A61F 13/08

(54) **ORTHESE DE CONTENTION**
RESTRIKTIVE ORTHESE
RESTRICTIVE ORTHOSIS

(30) Priorité: 07.04.2008 FR 0801895
(43) Date de publication de la demande: 23.02.2011
(73) Titulaire: Gibaud, 42000 Saint Etienne (FR)
(72) Inventeur: BECKERS, Thomas, F-69630 Chaponost (FR); VALOIS, Christophe, F-01090 Montmerle (FR)
(74) Mandataire: Delorme, Nicolas
(86) Numéro de dépôt international: PCT/FR2009/050598
(87) Numéro de publication internationale: WO 2009/136063

(56) Documents cités:
- WO-A-2006/097156
- WO-A-2008/006227
- DE-A1- 3 614 913
- FR-A- 615 464
- FR-A- 787 294
- FR-A- 938 777
- FR-A- 1 128 435
- FR-A- 2 588 890
- FR-A- 2 805 459

## Description

La présente invention concerne une orthèse de contention notamment d'un membre inférieur.

Dans le présent document, le terme orthèse de contention désigne, de manière générale des collants, des bas ou des chaussettes dont le port est prescrit à des personnes qui souffrent d'insuffisance veineuse.

De manière générale, une orthèse de contention est un article réalisé dans un matériau élastique. De manière classique, une orthèse de contention est obtenue par tricotage d'une maille très serrée avec insertion de fils de trame fortement élastique qui vont donner à l'orthèse ses propriétés élastiques.

Sur le plan thérapeutique, les orthèses de contention apportent un service reconnu.

En revanche, il est souvent reproché aux orthèses de contention leur aspect peu attractif qui peut dissuader certaines personnes de les porter.

On connaît par le document FR 2 588 890 une orthèse de contention qui permet de créer l'illusion de finesse et de transparence. Par le document WO2008/006227, on connaît une orthèse de contention qui possède des moyens pour évaluer la tension. Le document W02006/097156 vise un bas de contention pourvu de motifs.

On peut préciser qu'il n'est pas difficile d'imprimer une orthèse de contention en vue de lui conférer un aspect plus attractif car, par essence, une orthèse de contention présente une grande élasticité ; les éventuelles impressions seraient donc déformées lorsque l'orthèse est portée.

Dans ce contexte, un but de la présente invention est de proposer une orthèse de contention qui présente un motif décoratif à sa surface.

L'invention concerne une orthèse de contention ayant une partie de pied et une partie de jambe, formées de mailles de fils de tricotage intégrant des fils élastiques de trame ; de plus, l'orthèse incorpore au niveau de sa partie de jambe au moins un fil de vanisage qui possède des caractéristiques distinctes de celle des fils de tricotage et des fils élastiques de trame, le fil de vanisage recouvrant sélectivement le fil de tricotage dans au moins une maille sur la face endroit de l'orthèse.

La base de l'invention est d'incorporer un fil supplémentaire par vanisage dans la partie de jambe d'une orthèse de contention. Le fil de vanisage qui recouvre sélectivement certaines mailles sur la face endroit de l'orthèse forme ainsi un motif de toute forme qui apparaîtra après tricotage ou après une opération, par exemple, de lavage ou de teinture.

Selon une possibilité, le fil de vanisage est constitué d'une matière différente de celle constituant les fils de tricotage et les fils élastiques de trame.

Selon une autre possibilité, le fil de vanisage possède des propriétés tinctoriales distinctes de celles des fils de tricotage.

Cela permet après une opération de teinture de faire ressortir un motif formé par le fil de vanisage.

Selon une possibilité, le fil de vanisage est un fil de polyamide.

De plus, les fils élastiques de trame sont des fils d'élasthanne guipés de polyamide et/ou coton et les fils de tricotage sont des fils d'élasthanne guipés de polyamide.

En pratique, le fil de vanisage recouvre le fil de tricotage sur des aiguilles sélectionnées pour réaliser un motif sur la face externe de l'orthèse.

Pour sa bonne compréhension, l'invention est décrite en référence au dessin ci annexé.

Figure 1 représente une orthèse de contention selon l'invention,

Figure 2 représente l'entrelacement du tricot dans une partie de jambe de l'orthèse de la figure 1.

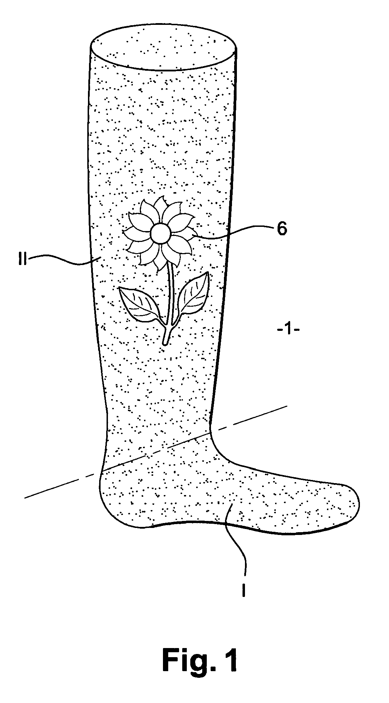

La figure 1 montre une orthèse de contention 1 élastique qui applique une pression dégressive sur le membre inférieur du patient. De façon schématique, cette orthèse permet de faire remonter le sang vers le cceur car elle exerce une pression plus forte à la cheville qu'au niveau du mollet ou de la cuisse.

L'orthèse de contention possède une partie de pied I tans laquelle s'insère le pied du patient et une partie de jambe II formant jambe qui s'étend jusqu'au mollet, jusqu'à la cuisse ou jusqu'à l'abdomen selon les cas. La partie de pied I couvre la partie du membre inférieur qui va de la région des malléoles jusqu'à l'extrémité des orteils tandis que la partie de jambe II est une partie tubulaire dont la section augmente en direction de la partie haute de la jambe.

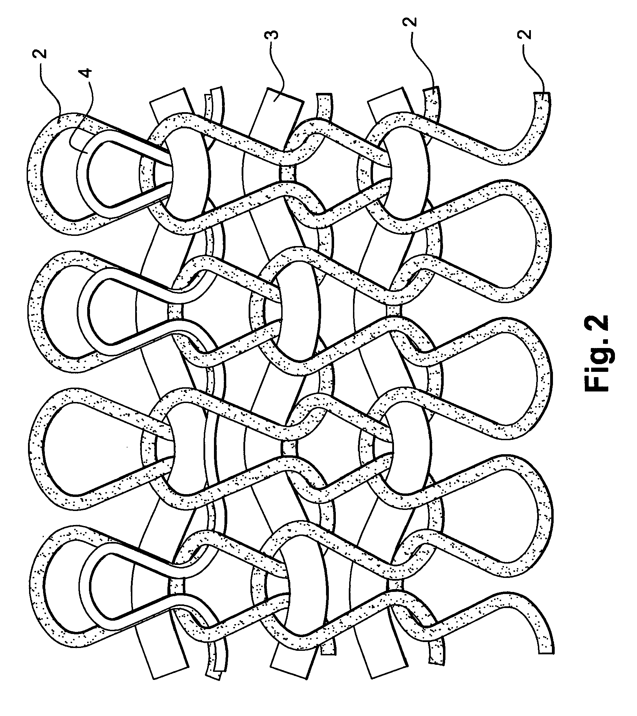

La partie de jambe II est donc apparente et c'est pour cela qu'il s'agit de la partie de l'orthèse qui possède la structure particulière de la figure 2.

La figure 2, de manière partielle, montre une forme d'exécution de l'invention ; sur cette figure, on voit que l'orthèse dans sa partie II est constituée de fil de tricotage 2 qui peuvent former des mailles jersey.

Ces fils de tricotage 2 peuvent être, par exemple, des fils de polyamide, d'élasthanne, guipés de polyamide, coton, etc.

Insérés dans les mailles jersey, on trouve des fils élastiques 3 qui donnent à l'orthèse sa propriété de contention. Ces fils élastiques 3 présentent une âme d'élasthanne avec un guipage de coton et/ou polyamide, par exemple. Selon la composition des fils élastiques 3, l'orthèse appartient à une classe thérapeutique spécifique.

De plus, dans sa partie de jambe II, l'orthèse intègre un fil de vanisage 4, c'est-à-dire un fil supplémentaire qui est tricoté avec un fil de tricotage 2 selon le motif que l'on cherche à obtenir. Ainsi, le fil de vanisage peut apparaître sur l'endroit de l'orthèse. La figure 2 fait apparaitre le fil de vanisage 4 au niveau de trois mailles dans lesquelles le fil de vanisage recouvre le fil de tricotage 2.

L'orthèse est de préférence fabriquée sur un métier circulaire et le fil de vanisage 4 est pris par des aiguilles sélectionnées selon le dessin 6 qui doit être obtenu.

Le fil de vanisage 4 a la particularité de présenter des caractéristiques différentes de celles des autres fils constituant l'orthèse.

Dans le cas où les fils de vanisage présentent une affinité tinctoriale différente de celle des autres fils, c'est-à-dire présenter des propriétés de fixation de couleur différente de celle des autres fils, il peut être prévu, après tricotage, une opération de teinture ou de lavage au cours de laquelle la couleur est retenue de manière différente par les fils de vanisage 4 et les autres fils 2 et 3 entrant dans la composition de l'orthèse. Ceci permet alors de faire apparaître un motif formé par les fils de vanisage.

On peut utiliser d'autres propriétés des fils de vanisage pour faire apparaître un motif après tricotage ; ainsi le fil de vanisage 4 peut par exemple :
- présenter une couleur différente de celle des autres fils de l'orthèse,
- présenter une matière différente de celle des autres fils de l'orthèse.

Les fils de vanisage prennent alors une couleur différente de celle des autres fils 2 et 3 de l'orthèse.

Les zones de la partie de jambe II de l'orthèse où les boucles de fil de vanisage se superposent aux boucles de fil de tricotage apparaissent dans une nuance de couleur ou de matière distinctes de celle du reste de l'orthèse qui est formée par les mailles de fils de tricotage 2. C'est ainsi que sont formés les motifs ou dessin 6 qui apparaissent sur la partie de jambe de l'orthèse.

Les fils de vanisage 4 n'ont pas d'impact sur la contention ou sur le confort de l'orthèse. Les fils de vanisage 4 ont comme fonction, de ponctuellement, recouvrir les fils de tricotage 2 et, grâce à leurs propriétés d'aspect de ressortir de manière différente des autres fils 2 et 3. Les fils de vanisage peuvent donc être par exemple de fils de polyester, polyamide, etc.

Bien entendu, l'invention n'est pas limitée à la forme de réalisation décrite ci-dessus à titre d'exemple non limitatif mais elle en embrasse au contraire toutes les formes de réalisation. Ainsi, la maille jersey montrée à la figure 2 pourrait être remplacée par une autre maille connue.

## Revendications

1. Orthèse de contention ayant une partie de pied chaussante et une partie de jambe II formées de mailles de fils de tricotage (2) intégrant des fils élastiques (3) de trame, **caractérisée en ce que** de plus, l'orthèse incorpore au niveau de sa partie de jambe II au moins un fil de vanisage (4) qui possède des caractéristiques distinctes de celles des fils de tricotage (2) et des fils élastiques (3) de trame, le fil de vanisage (4) recouvrant sélectivement le fil de tricotage (2) dans au moins une maille sur la face endroit de l'orthèse, et le fil de vanisage (4) recouvrant le fil de tricotage sur des aiguilles sélectionnées pour réaliser un motif sur la face externe de l'orthèse.

2. Orthèse selon la revendication 1, **caractérisée en ce que** le fil de vanisage (4) est constitué d'une matière différente de celle constituant les fils de tricotage (2) et les fils élastiques (3) de trame.

3. Orthèse selon la revendication 1 ou la revendication 2, **caractérisée en ce que** le fil de vanisage (4) possède des propriétés tinctoriales distinctes de celles des fils de tricotage (2).

4. Orthèse selon l'une des revendications 1 à 3, **caractérisée en ce que** le fil de vanisage (4) est un fil de polyamide.

5. Orthèse selon l'une des revendications 1 à 4, **caractérisée en ce que** les fils élastiques (3) de trame sont des fils d'élasthanne guipés de polyamide et/ou coton.

6. Orthèse selon l'une des revendications 1 à 5, **caractérisée en ce que** les fils de tricotage (2) sont des fils d'élasthanne guipés de polyamide.

## Patentansprüche

1. Stützorthese mit einem Schuhfußteil und einem Beinteil II, die von Wirkfadenmaschen (2) gebildet wird, die elastische Schussfäden (3) integrieren, **dadurch gekennzeichnet, dass** die Orthese zusätzlich auf Ebene ihres einteils II mindestens einen eckfaden (4) inkorporiert, der Merkmale besitzt, die sich von denen der Wirkfäden (2) und der elastischen Schussfäden (3) unterscheiden, wobei der eckfaden (4) den Wirkfaden (2) bei mindestens einer Masche auf der rechten Seite der Orthese selektiv bedeckt, und der Deckfaden (4) den Wirkfaden auf Nadeln bedeckt, die ausgewählt sind, um ein Motiv auf der Außenseite der Orthese zu realisieren.

2. Orthese nach Anspruch 1, **dadurch gekennzeichnet, dass** der Deckfaden (4) aus einem anderen Material als dem, das die Wirkfäden (2) und die elastischen Schussfäden (3) bildet, besteht.

3. Orthese nach Anspruch 1 oder Anspruch 2, **dadurch gekennzeichnet, dass** der Deckfaden (4) Farbmerkmale besitzt, die sich von denen der Wirkfäden (2) unterscheiden.

4. Orthese nach einem der Ansprüche 1 bis 3, **dadurch gekennzeichnet, dass** der eckfaden (4) ein Polyamidfaden ist.

5. Orthese nach einem der Ansprüche 1 bis 4, **dadurch gekennzeichnet, dass** die elastischen Schussfäden (3) Guipure-Elasthanfäden aus Polyamid und/oder Baumwolle sind.

6. Orthese nach einem der Ansprüche 1 bis 5, **dadurch gekennzeichnet, dass** die Wirkfäden (2) Guipure-Elasthanfäden aus Polyamid sind.

## Claims

1. A restrictive orthosis having a shoe foot portion and a leg portion II formed with stitches of knitting yarns (2) integrating elastic weft yarns (3), **characterized in that** additionally the orthosis incorporates at its leg portion II at least one plaiting yarn (4) which has characteristics distinct from those of the knitting yarns (2) and of the elastic weft yarns (3), the plaiting yarn (4) selectively covering the knitting yarn (2) in at least one stitch on the front face of the orthosis, and the plaiting yarn (4) covering the knitting yarn on selected needles in order to produce a pattern on the external face of the orthosis.

2. The orthosis according to claim 1, **characterized in that** the plaiting yarn (4) consists of a different material from the one forming the knitting yarns (2) and the weft elastic yarns (3).

3. The orthosis according to claim 1 or claim 2, **characterized in that** the plaiting yarn (4) has distinct tinctorial properties from those of the knitting yarns (2).

4. The orthosis according to one of claims 1 to 3, **characterized in that** the plaiting yarn (4) is a polyamide yarn.

5. The orthosis according to one of claims 1 to 4, **characterized in that** the weft elastic yarns (3) are elasthane yarns sheathed with polyamide and/or cotton.

6. The orthosis according to one of claims 1 to 5, **characterized in that** the knitting yarns (2) are elasthane yarns sheathed with polyamide.
